# EUROPÄISCHE PATENTANMELDUNG

(11) **EP 0 538 685 A2**
(43) Veröffentlichungstag der Anmeldung: **28.04.1993**
(21) Anmeldenummer: 92117238.3
(22) Anmeldetag: 09.10.1992
(51) Int. Cl.: C07C 69/708, C07C 67/31

(54) **Verfahren zur Herstellung von Vinyloxycarbonsäureestern**

(30) Priorität: 22.10.1991 DE 4134806
(71) Anmelder: BASF Aktiengesellschaft, D-67063 Ludwigshafen (DE)
(72) Erfinder: Karcher, Michael, Dr., W-6830 Schwetzingen (DE); Henkelmann, Jochem, Dr., W-6700 Ludwigshafen (DE)

(57) **Zusammenfassung**

Verfahren zur Herstellung von Vinyloxycarbonsäureestern der allgemeinen Formel I 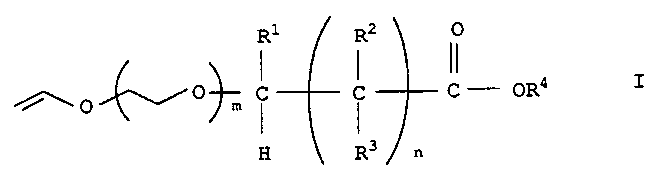
in der die Reste R¹, R² und R³ gleich oder verschieden sind und Wasserstoff oder eine C₁- bis C₂₀-Alkylgruppe bedeuten,
der Rest R⁴ eine unsubstituierte oder eine Hydroxygruppe tragende C₁- bis C₂₀-Alkylgruppe, eine unsubstituierte oder eine mit unter den Reaktionsbedingungen inerten Substituenten substituierte C₆- bis C₁₀-Arylgruppe oder C₇- bis C₁₂-Aralkylgruppe ist und in der
die Indices m und n gleich oder verschieden sind und für eine ganze Zahl von 0 bis 20 stehen, durch die Umsetzung eines Hydroxycarbonsäureesters der allgemeinen Formel II 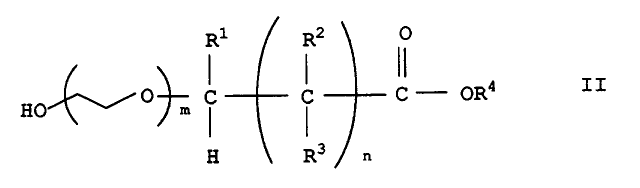
in der R¹, R², R³, R⁴, m und n die obengenannte Bedeutung haben, mit einem Vinylether der allgemeinen Formel III 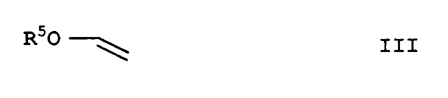
in der der Rest R⁵ eine C₁- bis C₂₀-Alkyl- oder eine C₃- bis C₈-Cycloalkylgruppe ist, in Gegenwart eines Katalysators, indem man als Katalysator für diese Umsetzung eine oder mehrere Palladiumverbindungen verwendet.

## Beschreibung

Die vorliegende Erfindung betrifft ein Verfahren zur Herstellung von Vinyloxycarbonsäureestern der allgemeinen Formel I 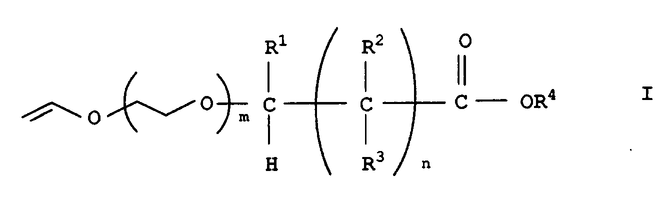
in der die Reste R¹, R² und R³ gleich oder verschieden sind und Wasserstoff oder eine C₁- bis C₂₀-Alkylgruppe bedeuten,
der Rest R⁴ eine unsubstituierte oder eine Hydroxygruppe tragende C₁- bis C₂₀-Alkylgruppe, eine unsubstituierte oder eine mit unter den Reaktionsbedingungen inerten Substituenten substituierte C₆- bis C₁₀-Arylgruppe oder C₇- bis C₁₂-Aralkylgruppe ist und in der
die Indices m und n gleich oder verschieden sind und für eine ganze Zahl von 0 bis 20 stehen, durch die Umsetzung eines Hydroxycarbonsäureesters der allgemeinen Formel II 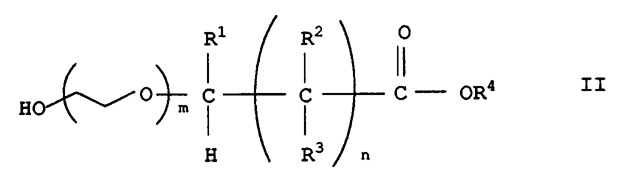
in der R¹, R², R³, R⁴, m und n die oben genannte Bedeutung haben, mit einem Vinylether der allgemeinen Formel III 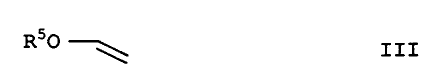
in der der Rest R⁵ eine C₁- bis C₂₀-Alkyl- oder eine C₃- bis C₈-Cycloalkylgruppe ist, in Gegenwart eines Katalysators.

Die Übertragung von Vinylgruppen aus Vinylgruppen-enthaltenden Verbindungen, insbesondere aus Vinylethern, auf Alkohole wird durch Quecksilber-, Cadmium- und Zinnsalze katalysiert (J. Org. Chem. 14, 1057 (1949)). Zur Herstellung von Vinyloxycarbonsäureestern durch die Übertragung einer Vinylgruppe aus einem Vinylether auf die betreffende Hydroxycarbonsäure wurden bislang ausschließlich Quecksilbersalze als Katalysatoren verwendet (US-A 2 579 411; Makromol. Chem. 126, 173 (1969); Ind. Eng. Chem. 50, 1703 (1958)).

McKeon et al (Tetrahedron 28, 233 (1972)) beschrieben die Vinylgruppenübertragung von Vinylethern auf Alkohole, wobei sie als Katalysatoren Diacetato-(1,20-phenanthrolin)- sowie Diacetato-(2,2'-bipyridyl)-palladium(II)-Komplexe als Katalysatoren verwenden.

Gemaß EP-A 351 603 werden Ruthenium-Verbindungen als Katalysatoren zur Übertragung der Vinylgruppe von Vinylestern auf Alkohole und Hydroxycarbonsäureester benutzt.

Da Vinylether von Hydroxycarbonsäurerestern für mannigfaltige Zwecke Anwendung finden, beispielsweise als Schmieröladditive, zur Herstellung von Schutzüberzügen (J. Am. Oil Chemists' Soc. 40, 143 (1963)), zur Herstellung von Polymeren, die als Wachsersatzstoff in Polituren und Appreturen dienen, sowie neuerdings zur Herstellung biologisch abbaubarer Polymere (Makromol. Chem., Rapid Commun. 9, 1 (1988)), Vinylether einfacher Alkohole als Vinyl-Quelle zur Umvinylierung in großen Mengen zur Verfügung stehen und die Herstellung von Vinylethern von Hydroxycarbonsäureestern durch die Umvinylierung von Vinylethern mit Hydroxycarbonsäureestern nach den bislang bekannten Methoden der quecksilber- und palladium-katalysierten Umvinylierung im industriellen Maßstab teils aus ökotoxikologischen, teils aus Gründen unzureichender Ausbeuten im industriellen Maßstab nicht möglich ist, lag der vorliegenden Erfindung die Aufgabe zugrunde, ein Verfahren zur Herstellung von Vinyloxycarbonsäureestern zu finden, das deren Herstellung wirtschaftlich, in guten Ausbeuten und Selektivitäten und auf umweltverträgliche Weise ermöglicht.

Dementsprechend wurde ein Verfahren zur Herstellung von Vinyloxycarbonsäureestern der allgemeinen Formel I 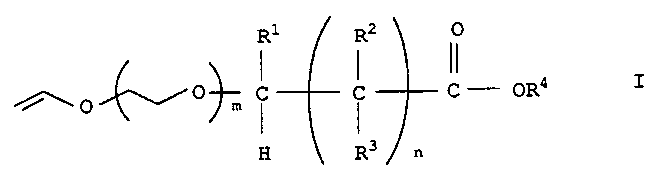
in der die Reste R¹, R² und R³ gleich oder verschieden sind und Wasserstoff oder eine C₁- bis C₂₀-Alkylgruppe bedeuten,
der Rest R⁴ eine unsubstituierte oder eine Hydroxygruppe tragende C₁- bis C₂₀-Alkylgruppe, eine unsubstituierte oder eine mit unter den Reaktionsbedingungen inerten Substituenten substituierte C₆- bis C₁₀-Arylgruppe oder C₇- bis C₁₂-Aralkylgruppe ist und in der
die Indices m und n gleich oder verschieden sind und für eine ganze Zahl von 0 bis 20 stehen, durch die Umsetzung eines Hydroxycarbonsäureesters der allgemeinen Formel II 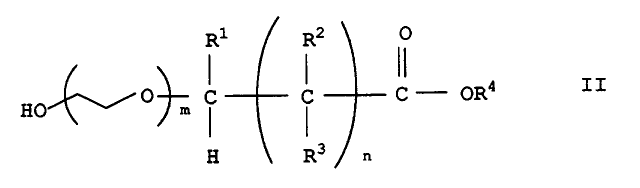
in der R¹, R², R³, R⁴, m und n die obengenannte Bedeutung haben, mit einem Vinylether der allgemeinen Formel III 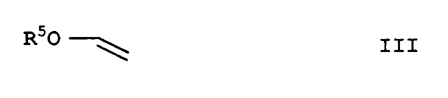
in der der Rest R⁵ eine C₁- bis C₂₀-Alkyl- oder eine C₃- bis C₈-Cycloalkylgruppe ist, in Gegenwart eines Katalysators, gefunden, das dadurch gekennzeichnet ist, daß man als Katalysator für diese Umsetzung eine oder mehrere Palladiumverbindungen verwendet.

Bei der dem erfindungsgemäßen Verfahren zugrundeliegenden chemischen Umsetzung wird also eine Vinylgruppe vom Vinylether III auf den Hydroxycarbonsäureester II übertragen, wobei als Katalysator Verbindungen des Palladiums verwendet werden. Das Palladium liegt in diesen Verbindungen im allgemeinen in der Oxidationsstufe +II vor.

Als Palladium-Verbindungen können die Salze oder Komplexe, insbesondere Chelatkomplexe, des Palladiums verwendet werden. Als solche Salze sind z.B. die Halogenide, insbesondere das Chlorid, das Sulfat, Nitrat oder Cyanid des Palladiums zu nennen, besonders bevorzugt werden jedoch die Carboxylate des Palladiums, insbesondere dessen C₁- bis C₁₀-Carboxylate, beispielsweise dessen Formiat, Acetat, Propionat, Butyrat, Valerianat, Isovalerianat, Pivaloat, Decanoat, Succinat, Adipat, Acetoacetat und Benzoat eingesetzt. Unter diesen Carboxylaten des Palladiums sind das Acetat und das Acetoacetat besonders bevorzugt.

Vorzugsweise werden die im erfindungsgemäßen Verfahren eingesetzten Palladium-Verbindungen, insbesondere die Palladiumcarboxylate, in Form von Chelatkomplexen benutzt, wobei stickstoffhaltige Chelatliganden, wie 2,2-Bipyridin, 1,8-Naphthyridin oder Phenanthrolin, die mit unter den Reaktionsbedingungen inerten Substituenten substituiert sein können, bevorzugt sind. Vorteilhaft können aber auch Chelatkomplexe des Palladiums eingesetzt werden, welche stickstoff-freie Chelatliganden, insbesondere Chelatliganden des Pentandiontyps, wie Acetylaceton, enthalten. Als Beispiele für solche in Form von Chelatkomplexen als Katalysatoren dienende Palladium-Verbindungen seien Phenanthrolinpalladiumdichlorid, 2,2-Bipyridinpalladiumdichlorid, 2,2-Bipyridinpalladiumdibromid, Phenanthrolinpalladiumdiacetat, 2 ,2-Bipyridiumpalladiumdiacetat, Phenanthrolinpalladiumdinitrat, Palladiumacetylacetonatodichlorid, Palladiumacetylacetonatodiacetat, Palladiumacetylacetonatodipropionat, Palladiumacetylacetonatosulfat und Palladiumacetylacetonatodicyanid genannt.

Die genannten Palladiumkomplex-Katalysatoren lösen sich homogen im Reaktionsmedium, so daß die Umsetzung homogenkatalytisch durchgeführt werden kann. Es ist aber auch möglich, im erfindungsgemäßen Verfahren heterogenisierte Palladiumkomplex-Katalysatoren zu verwenden, insbesondere solche, in denen das Palladium an polymere Matrizes fixiert ist. Solche polymeren Matrizes können Harze wie Styrol-divinylbenzol-Harze oder Phenol-Formaldehyd-Harze sein, an die die betreffenden Chelatliganden gebunden sind, welche wiederum mit den Palladium-Verbindungen Komplexe bilden und diese derart quasi immobilisieren. Als heterogene Matrizes zur Immobilisierung der Palladiumkomplexe können auch anorganische Trägermaterialien nach der Hydrophobisierung und chemischen Modifizierung ihrer Oberflächen mittels organischen Reagentien dienen. Solche heterogenisierten, polymergebundenen Platinmetallkomplex-Katalysatoren sind beispielsweise nach dem Verfahren von Zhuangyu et al (Reactive Polymers 9, 249 (1988)) erhältlich. Der Vorteil der Anwendung solcher heterogenisierter Katalysatoren liegt insbesondere in der leichteren und schonenderen Abtrennbarkeit des Katalysators vom Reaktionsgut. Dieser kann in einem Festbett angeordnet und von der Reaktionsmischung durchströmt werden oder aber auch im Reaktionsgut suspendiert und nach beendeter Umsetzung mechanisch abgetrennt werden.

Werden hingegen homogen im Reaktionsmedium gelöste Palladium-Verbindungen verwendet, so können diese nach beendeter Umsetzung vom Reaktionsprodukt z.B. mittels physikalischer Trennverfahren, beispielsweise durch Adsorption an geeignete Adsorbentien, insbesondere anorganische Adsorbentien oder Kationenaustauscher, abgetrennt werden. Als anorganische Adsorbentien seien beispielhaft Aktivkohle, Calciumcarbonat, Bariumsulfat, Siliciumdioxid, Aluminiumoxide, Titandioxide und Zirkoniumdioxid genannt. Als Kationenaustauscher können handelsübliche Kationenaustauscherharze verwendet werden. Eine weitere Möglichkeit der Trennung von Produkt und Katalysator besteht in der destillativen Abtrennung des Reaktionsproduktes, die gewünschtenfalls unter vermindertem Druck durchgeführt und in Kolonnen oder vorzugsweise in Verdampfern, insbesondere Dünnfilmverdampfern, vorgenommen werden kann, wobei gewünschtenfalls zur zusätzlichen Verminderung der thermischen Belastung von Produkt und Katalysator zusätzlich noch mit einem Inertgas, wie Stickstoff oder Argon, gestrippt werden kann. Bei einer derartigen destillativen Abtrennung des Reaktionsproduktes kann die Anwesenheit eines hochsiedenden Lösungsmittels im Reaktionsprodukt von Vorteil sein, insbesondere kann der im hochsiedenden Lösungsmittel zurückbleibende Katalysator wieder in die Umsetzung zurückgeführt und wiederverwendet werden. Als hochsiedende Lösungsmittel eignen sich zu diesem Zweck insbesondere Sulfoxide und Sulfone, wie Dimethylsulfoxid, Dimethylsulfon und Sulfolan (Tetrahydrothiophen-1,1-dioxid) sowie hochsiedende, oligomere Ethylenglykol-, Propylenglykol- und 1,4-Butylenglykolether.

Die Palladiumverbindungen werden im allgemeinen in Mengen von 0,01 bis 5 mol-%, insbesondere von 0,1 bis 4 mol-% und besonders bevorzugt in Mengen von 0,2 bis 0,1 mol-%, bezogen auf den Hydroxycarbonsäureester II, eingesetzt. Die Anwendung höherer oder geringerer Katalysatormengen ist möglich, in der Regel jedoch nicht bevorzugt. Die Palladiumkomplex-Katalysatoren können als solche zum Reaktionsgemisch gegeben werden, sie können vorteilhaft aber auch in situ im Reaktionsgemisch aus den einzelnen Komponenten, Palladiumsalz und Chelatligand, erzeugt werden.

Da die erfindungsgemäße Umsetzung eine Gleichgewichtsreaktion ist, wird zur Erzielung eines möglichst vollständigen Umsatzes zweckmäßigerweise einer der beiden Reaktanten, üblicherweise der Vinylether III, im Überschuß eingesetzt. Im allgemeinen wird der Vinylether III in einem 1,1- bis 10-fachen molaren Überschuß, vorzugsweise in einem 2- bis 5-fachen molaren Überschuß, bezüglich des Hydroxycarbonsäureesters II, verwendet. Der überschüssige Vinylether III kann nach beendeter Umsetzung wiedergewonnen werden, beispielsweise bei der destillativen Aufarbeitung des Reaktionsproduktes und gewünschtenfalls in die Umsetzung zurückgeführt werden.

Die Umsetzung wird im allgemeinen bei Temperaturen von -40 bis +150^{o}C, vorzugsweise bei 0 bis 100^{o}C und besonders bevorzugt bei 20 bis 80^{o}C, unter Atmosphärendruck oder unter erhöhtem Druck, insbesondere unter dem Eigendruck des Reaktionssystems vorgenommen. Das Arbeiten unter vermindertem Druck ist ebenfalls möglich.

Die Umetherung kann in Gegenwart eines unter den Reaktionsbedingungen inerten Lösungsmittels, beispielsweise einem Ether, wie Diethylether, Diisopropylether, Methyl-tert.-butylether, Tetrahydrofuran, einem Keton, wie Acetophenon, Kohlenwasserstoffen, wie Benzol, Toluol oder Cyclohexan, sterisch gehinderten Alkoholen, wie tert.-Butanol oder Neopentanol, einem Formamid, wie N,H-Dimethylformamid oder N,N-Diethylformamid, einem Lactam, wie N-Methylpyrrolidon, einem cyclischen Harnstoff, wie N, N' -Dimethylethylenharnstoff oder N,N'-Dimethylpropylenharnstoff, einem Sulfoxid, wie Dimethylsulfoxid oder einem Sulfon, wie Dimethylsulfon oder Sulfolan erfolgen. Ist mindestens einer der beiden Reaktanten unter den Reaktionsbedingungen flüssig, so kann die Umsetzung vorteilhaft auch in Abwesenheit eines Lösungsmittels durchgeführt werden.

Als Ausgangsmaterialien zur Herstellung der Vinyloxycarbonsäureester I dienen die Hydroxycarbonsäureester der allgemeinen Formel II 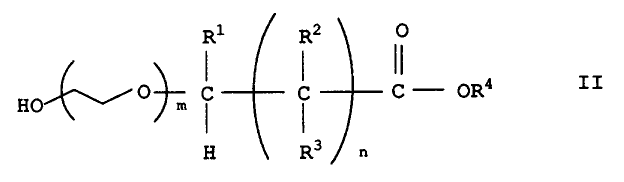
in der die Reste R¹, R² und R³ gleich oder verschieden sind und Wasserstoff oder eine geradkettige oder verzweigte C₁- bis C₂₀-Alkylgruppe bedeuten. Bevorzugte Reste R¹, R² und R³ sind Wasserstoff und C₁- bis C₄-Alkylgruppen. An die Hydroxygruppe des Hydroxycarbonsäureesters können gegebenenfalls noch 1 bis 20 Oxyethyleneinheiten ankondensiert sein, so daß der Index m eine ganze Zahl von 0 bis 20 bedeutet. Je nach Länge und Verzweigungsgrad des verwendeten Hydroxycarbonsäureesters II kann der Index n ebenfalls eine ganze Zahl von 0 bis 20 sein.

Bevorzugt werden Hydroxycarbonsäureester der Glykolsäure, Milchsäure, Hydroxypivalinsäure und Hydroxypropionsäure verwendet. Weitere bevorzugte Hydroxycarbonsäureester sind solche von Hydroxycarbonsäuren, welche von einfach ungesättigten Fettsäuren, wie Crotonsäure, Tiglinsäure, Palmitoleinsäure, Ölsäure oder Erucasäure, abgeleitet und beispielsweise durch die säure-katalysierte Addition von Wasser an deren Doppelbindungen leicht und in großer Menge erhältlich sind. Bevorzugte Ausgangsmaterialien sind außerdem Hydroxycarbonsäureester von Hydroxycarbonsäuren, die durch die Hydrierung von ungesättigten Hydroxyfettsäuren, wie Ricinolsäure oder Kamalolensäure, leicht und in großer Menge zugänglich sind (vgl. Makromol. Chem. 126, 173 (1969)).

Der Rest R⁴ der Hydroxycarbonsäureester kann eine geradkettige oder verzweigte C₁- bis C₂₀-Alkylgruppe, welche gewünschtenfalls weitere Hydroxylgruppen, vorzugsweise nicht mehr als eine Hydroxylgruppe, tragen kann oder eine unsubstituierte oder mit unter den Reaktionsbedingungen inerten Substituenten wie C₁- bis C₄-Alkyl-, C₁- bis C₄-Alkoxy-, Halogen-, Nitro- oder Nitrilogruppen, substituierte C₆- bis C₁₀-Arylgruppe oder C₇- bis C₁₂-Aralkylgruppe sein. Vorzugsweise ist der Rest R⁴ eine unsubstituierte C₁- bis C₄-Alkylgruppe. Als Aryl- oder Aralkylreste R⁴ werden vorzugsweise unsubstituierte Aryl- oder Aralkylreste verwendet. Ist R⁴ eine substituierte Aryl- oder Aralkylgruppe, so werden solche Aryl- oder Aralkylgruppen bevorzugt, die nicht mehr als 3 Substituenten tragen.

Die Hydroxycarbonsäureester II können z.B. durch Umesterung der in natürlichen Fetten enthaltenen Hydroxycarbonsäuren mit dem dem Rest R⁴ entsprechenden Alkohol unter Alkoholat-Katalyse erhalten werden. Synthetisch hergestellte Hydroxycarbonsäuren können z.B. nach dem Verfahren von Swern et al (J. Am. Chem. Soc. 67, 902 (1945) mit dem betreffenden Alkohol verestert werden. Hydroxypivalinsäureester können z.B. durch die elektrochemische Oxidation von Hydroxypivalaldehyd in alkoholischer Lösung (J. Org. Chem. 53, 218 (1988)) oder nach dem Verfahren gemäß DE-A 12 80 237 erzeugt werden. Hydroxypivalinsäureneopentylglykolester ist durch die Disproportionierung von Hydroxypivalaldehyd, z.B. gemäß EP-A 420 770, zugänglich. Glykolsäuren können nach dem Verfahren von US-A 3 799 977 durch die Oxidation von Ethylenglykol oder von Oligoethylenglykolen hergestellt und auf herkömmliche Weise verestert werden. Als eine alternative Herstellungsmethode, insbesondere für die Säuren mit längerkettigen Oligoethylenglykolresten, ist das Verfahren gemäß Synthesis 42 (1981) anwendbar. Milchsäureester können mit Hilfe von Milchsäurebakterien durch die Vergärung von Zucker und anschließende Veresterung der gebildeten Milchsäure gewonnen werden.

Zur Herstellung der Vinyloxycarbonsäureester I wird der Hydroxycarbonsäureester II mit einem Vinylether III 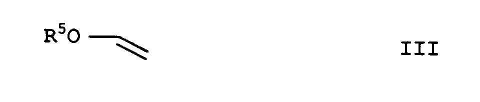
umgesetzt, in der der Rest R⁵ eine geradkettige oder verzweigte C₁- bis C₂₀-Alkylgruppe oder eine C₃- bis C₈-Cycloalkylgruppe sein kann. Vorzugsweise werden Vinylether III verwendet, deren Rest R⁵ eine C₁- bis C₆-Alkylgruppe ist. Diese Vinylether sind auf einfache Weise durch die Reppe-Vinylierung der betreffenden Alkohole mit Acetylen in Gegenwart starker Basen zugänglich (vgl. Liebigs Ann. Chem. 601, 81 (1956)).

Nach dem erfindungsgemäßen Verfahren können die Vinyloxycarbonsäureester I wirtschaftlich, umweltverträglich, in guten Ausbeuten und mit exzellenten Selektivitäten erhalten werden. Die befürchtete Abscheidung des Palladiums an den Wandungen der Reaktionsapparate spielen bei den erfindungsgemäß angewandten Reaktionsbedingungen praktisch keine Rolle.

### Beispiele

### Beispiel 1: Vinyloxypivalinsäuremethylester

211,6 g (1,6 mol) Hydroxypivalinsäuremethylester und 800 g (8 mol) Vinylisobutylether wurden mit 3 g (7,4 mmol) Phenanthrolinpalladiumdiacetat versetzt. Das Gemisch wurde auf 70^{o}C erwärmt und 8 h bei dieser Temperatur gerührt. Nach 8 h Reaktionszeit betrug der Umsatz 70 %. Nebenprodukte konnten gaschromatographisch in der Reaktionsmischung nicht detektiert werden.

Die Reaktionsmischung wurde destillativ aufgearbeitet.
Ausbeute: 177 g, entsprechend 70 % Ausbeute, entsprechend
100 % Selektivität
Sdp.: 45^{o}C/7 mbar
¹H-NMR (CDCl₃; 250 MHz): σ = 6,4 (dd,1H); 4,15 (m, 1H); 3,95 (m, 1H); 3,7 (s, 2H); 3,65 (s, 3H); 1,2 (s, 6H).

### Beispiel 2: Vinyloxyglykolsäureethylester

10 g (96 mmol) Glykolsäureethylester, 69 g (0,96 mol) Ethylvinylether sowie 1,5 g (3,7 mmol) Phenanthrolinpalladiumdiacetat wurden 33 h bei Raumtemperatur gerührt. Die Reaktionsmischung wurde mit kalter Natriumcarbonat-Lösung und Wasser extrahiert und nach Trocknung über Magnesiumsulfat destilliert.

Ausbeute: 45 %; Selektivität: 90 %
Sdp. : 51^{o}C/12 mbar
¹H-NMR (CDCl₃; 250 MHz): σ = 6,45 (dd, 1H); 4,3 (s, 2H); 4,1-4,25 (m, 3H); 4,05 (m, 1H); 1,3 (t, 3H)

### Beispiel 3: Vinyloxymilchsäureester

10,4 g (0,1 mol) Milchsäuremethylester, 50 g (0,5 mol) Vinylisobutylether und 0,5 g (1,2 mmol) Phenanthrolinpalladiumdiacetat wurden 6 h auf 60^{o}C erwärmt. Bei einer Selektivität von 100 % betrug der Umsatz 25 %. Nach Extraktion der Reaktionsmischung mit Natriumcarbonatlösung und Wasser wurde diese destillativ aufgearbeitet
Sdp.: 25^{o}C/2 mbar
¹H-NMR (CDCl₃, 250 MHz): σ = 6,4 (dd, 1H); 4,42 (q, 1H); 4,25 (m, 1H); 4,1 (m,1H); 3,78 (s,3H); 1,5 (d,3H)

### Beispiel 4

122,4 g (0,6 mol) Hydroxypivalinsäureneopentylglykolester (HPN) wurden mit 600 g (6 mol) Vinylisobutylether und 1,2 g (2,9 mmol) Phenanthrolinpalladiumdiacetat 10 h bei 70^{o} C gerührt. Nach dieser Zeit waren 88 % HPN umgesetzt. Die Selektivität bezüglich die Divinylethers betrug 57 %, bezüglich der beiden Stellungsisomeren Monovinylether 43 %. Die Reaktionsmischung wurde destillativ aufgearbeitet.
Sdp.: 95^{o}C/2 mbar (ca. 80 %iges Produkt).

### Beispiel 5

142,6 g (0,84 mol) 2-Hydroxyethoxyessigsäuremethylester wurden mit 250 g (2,5 mol) Vinylisobutylether und 3 g (7,25 mmol) Phenanthrolinpalladiumdiacetat 10 h bei 50^{o}C gerührt. Es wurden bei einer Selektivität von 98 % ein Umsatz von 55 % erzielt.

Der überschüssige Vinylisobutylether wurde abdestilliert, der Destillationssumpf mit Wasser verdünnt und mehrfach mit Methyl-tert. -butylether extrahiert. Die vereinigten Extrakte wurden destillativ aufgearbeitet.
Sdp.: 87^{o}C/2 mbar.

## Patentansprüche

1. Verfahren zur Herstellung von Vinyloxycarbonsäureestern der allgemeinen Formel I 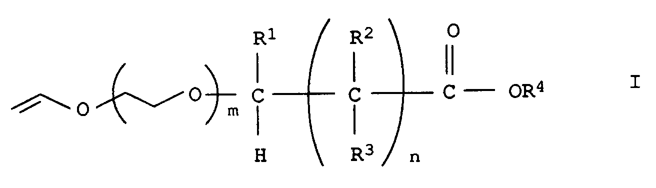 in der die Reste R¹, R² und R³ gleich oder verschieden sind und Wasserstoff oder eine C₁- bis C₂₀-Alkylgruppe bedeuten,
der Rest R⁴ eine unsubstituierte oder eine Hydroxygruppe tragende C₁- bis C₂₀-Alkylgruppe, eine unsubstituierte oder eine mit unter den Reaktionsbedingungen inerten Substituenten substituierte C₆- bis C₁₀-Arylgruppe oder C₇- bis C₁₂-Aralkylgruppe ist und in der
die Indices m und n gleich oder verschieden sind und für eine ganze Zahl von 0 bis 20 stehen, durch die Umsetzung eines Hydroxycarbonsäureesters der allgemeinen Formel II 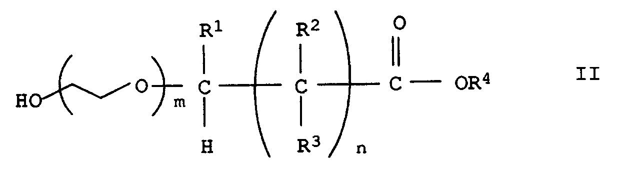 in der R¹, R², R³, R⁴, m und n die obengenannte Bedeutung haben, mit einem Vinylether der allgemeinen Formel III 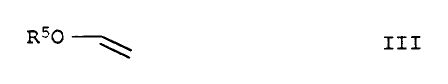 in der der Rest R⁵ eine C₁- bis C₂₀-Alkyl- oder eine C₃- bis C₈-Cycloalkylgruppe ist, in Gegenwart eines Katalysators, dadurch gekennzeichnet, daß man als Katalysatoren für diese Umsetzung eine oder mehrere Palladiumverbindungen verwendet.

2. Verfahren nach Anspruch 1, dadurch gekennzeichnet, daß man die Umsetzung bei -40 bis 150^{o}C durchführt.

3. Verfahren nach Anspruch 1, dadurch gekennzeichnet, daß man als Katalysatoren durch Chelat- oder Carboxylatliganden stabilisierte Verbindungen des Palladiums einsetzt.

4. Verfahren nach Anspruch 1, dadurch gekennzeichnet, daß man als Katalysatoren mit Chelatliganden stabilisierte Carboxylate des Palladiums verwendet.

5. Verfahren nach Anspruch 1, dadurch gekennzeichnet, daß man polymere Katalysatoren einsetzt, in denen die Palladiumverbindung durch an Polymermatrizes fixierte Chelatliganden komplexiert sind.

6. Verfahren nach Anspruch 1, dadurch gekennzeichnet, daß man die Palladiumverbindung in Mengen von 0,01 bis 5 mol-%, bezogen auf den Hydroxycarbonsäureester II, einsetzt.
